(19) Europäisches Patentamt
European Patent Office
Office européen des brevets

(11) Veröffentlichungsnummer: **0 230 557 A2**

# (12) EUROPÄISCHE PATENTANMELDUNG

(21) Anmeldenummer: **86116493.7**

(22) Anmeldetag: **27.11.86**

(51) Int. Cl.4: **C07D 493/04**, A61K 31/34, //(C07D493/04,307:00,307:00)

(30) Priorität: **24.01.86 DE 3602067**

(43) Veröffentlichungstag der Anmeldung: **05.08.87 Patentblatt 87/32**

(84) Benannte Vertragsstaaten: **AT BE CH DE ES FR GB GR IT LI LU NL SE**

(71) Anmelder: **Heinrich Mack Nachf.**
**Postfach 2064**
**D-7918 Illertissen(DE)**

(72) Erfinder: **Stoss, Peter, Dr. Dipl.-Chem.**
**Mozartstrasse 15**
**D-7918 Illertissen(DE)**
Erfinder: **Leitold, Matyas, Dr.**
**Klauflügelweg 21**
**D-7950 Biberach(DE)**

(74) Vertreter: **Lederer, Franz, Dr. et al**
**Vanderwerth, Lederer & Riederer**
**Patentanwälte Lucile-Grahn-Strasse 22**
**D-8000 München 80(DE)**

## (54) 2,6-Dioxabicyclo[3.3.0]octan-Derivate, ihre Herstellung und Verwendung als Arzneimittel.

(57) Die Erfindung betrifft neue 2,6-Dioxabicyclo[3.3.0] octan-Derivate der allgemeinen Formel I

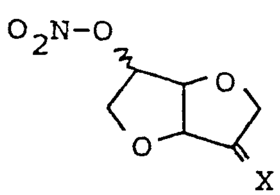

I

worin X die in der Beschreibung angegebene Bedeutung besitzt, Verfahren zu ihrer Herstellung und ihre Verwendung als Arzneimittel, insbesondere zur Vorbeugung und Bekämpfung von Herz-und Kreislauferkrankungen.

EP 0 230 557 A2

## 2,6-Dioxabicyclo[3.3.0]octan-Derivate, ihre Herstellung und Verwendung als Arzneimittel

2,6-Dioxabicyclo[3.3.0]octan-4,8-diole und deren Derivate, besser bekannt unter der Bezeichnung 1,4:3,6-Dianhydrohexite oder Isohexide, stellen eine wiederholt bearbeitete Verbindungsklasse dar. Dagegen liegen über davon abgeleitete Oxidationsprodukte, also Mono-und Diketone von 2,6-Dioxabicyclo[3.3.0]-octanen, bisher kaum Veröffentlichungen vor.

Ein Bericht in J. Am. Chem. Soc. 68, 939 (1946) postuliert zwar derartige Ketone als hypothetische Zwischenprodukte, ohne diese jedoch zu isolieren und zu charakterisieren. Das erfolgt dann erstmals in Chem. Ber. 96, 3195 (1963), sowie in einer dazu korrespondierenden deutschen Patentschrift DE 1 443 385. Eine weitere Erwähnung des Diketons findet sich in Carbohyd. Res. 11, 199 (1969). Kürzlich wurde noch eine europäische Patentanmeldung EP 125 986, bekannt, in der die Herstellung der erwähnten Ketone durch anodische Oxidation der entsprechenden sekundären Alkohole beschrieben wird. Des gleichen Verfahrens bedienen sich die Autoren in Biomass 6, 193 (1985).

Sämtliche genannte Publikationen beziehen sich ausschließlich auf die Grundkörper, d.h. die unsubstituierten Ketone, nämlich
8-exo-Hydroxy-2,6-dioxabicyclo[3.3.0]octan-4-on (= 1,4:3,6-Dianhydro-L-sorbose),
8-endo-Hydroxy-2,6-dioxabicyclo[3.3.0]octan-4-on (= 1,4:3,6-Dianhydro-D-fructose)
und
2,6-Dioxabicyclo[3.3.0]octan-4,8-dion (= 1,4:3,6-Dianhydro-D-threo-2,5-hexodiulose).

Monoketone, bei denen die noch im Molekül vorhandene OH-Gruppe substituiert ist, sowie an der Ketofunktion derivatisierte Verbindungen, sind, mit Ausnahme der zur Charakterisierung herangezogenen 2,4-Dinitrophenylhydrazone, bisher nicht bekannt. Ebenso liegen keinerlei Informationen über die therapeutische Verwertbarkeit solcher Verbindungen vor.

Mit den erfindungsgemäßen Verbindungen werden neuartig strukturierte Moleküle zur Verfügung gestellt, die sich überraschenderweise durch wertvolle pharmakologische Eigenschaften auszeichnen und somit einen Beitrag zur Bereicherung der therapeutischen Möglichkeiten liefern.

Gegenstand der Erfindung sind neue 2,6-Dioxabicyclo[3.3.0] octan-Derivate der allgemeinen Formel I

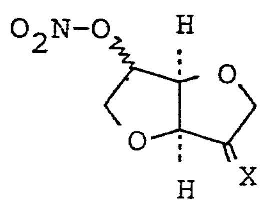

I

worin X für einen der nachfolgenden Substituenten steht:

O

$(OR^1)_2$,

wobei $R^1$ einen geradkettigen oder verzweigten Alkylrest mit 1 bis 20 Kohlenstoffatomen darstellt,

$$O-\underset{R^2}{CH}-(A)_m-CH_2-O,$$

worin $R^2$ Wasserstoff oder einen $-CH_2$-Hal, $-CH_2-OH$ oder $-CH_2ONO_2$-Rest, A einen $=CH_2$, $=CH$-Hal, $=CH-OH$ oder $=CH-ONO_2$-Rest und m die Zahlen 0 oder 1 bedeuten, wobei Hal für einen Halogenrest, insbesondere für einen Chlor-, Brom-oder Jodrest steht,

$N{-}OH$

$N{-}NH{-}R^3$

worin $R^3$ Wasserstoff oder einen der Reste

$C{=}Y,$

$\;|$

$NH_2$

wobei Y für O, S oder NH steht, oder
COOR⁴, wobei R⁴ für eine geradkettige oder verzweigte Alkylgruppe mit 1 bis 5 Kohlenstoffatomen steht, oder

$C\;=\;N,$

$|\quad\;\;|$

$NH{-}(CH_2)_n$

worin n die Zahlen 1 bis 4 bedeuten,
sowie gegebenenfalls deren Salze mit anorganischen und organischen Säuren, vorzugsweise mit pharmazeutisch akzeptablen Säuren.

In der allgemeinen Formel I kann die Bindung zwischen Ringsystem und der $O_2N$-O-Gruppe sowohl endo-als auch exo-ständig angeordnet sein. Dies ist in der Formel durch eine Wellenlinie ( ∿∿ ) zum Ausdruck gebracht.

Soweit nicht anders angegeben, ist eine Alkylgruppe eine solche mit 1-20, vorzugsweise 1-7 und insbesondere 1-3 Kohlenstoffatomen.

Unter den gegebenenfalls zum Erfindungsgegenstand gehörigen Salzen sind solche mit anorganischen und organischen Säuren zu verstehen, vorzugsweise jedoch solche mit pharmazeutisch akzeptablen Säuren. Beispiele hierfür sind Hydrochloride, Hydrobromide, Nitrate, Sulfate, Phosphate, Acetate, Oxalate, Maleate, Fumarate, Tartrate, Lactate, Malate, Malonate, Citrate, Salicylate, Methansulfonate, Benzolsulfonate, Toluolsulfonate und Naphthalinsulphonate.

Diese oder andere Salze der neuen Verbindungen, wie z.B. Pikrate, können gegebenenfalls auch zur Reinigung der freien Basen dienen, indem man die freie Base in ein Salz überführt, dieses abtrennt und gegebenenfalls umkristallisiert oder anderweitig reinigt und aus dem Salz wiederum die Base freisetzt.

2,6-Dioxabicyclo[3.3.0]octane bestehen aus zwei cis-verknüpften, nahezu ebenen Tetrahydrofuranringen, die einen Winkel von ca. 120° miteinander bilden. Je nach der Orientierung zum Ringsystem unterscheidet man zwischen endo-und exo-ständigen Substituenten. So sind z.B. von den eingangs zitierten 4,8-Diolen alle drei möglichen Isomeren bekannt, nämlich Isomannid (2 endo OH-Gruppen), Isoidid (2 exo OH-Gruppen) und Isosorbid ( 1 endo und 1 exo OH-Gruppe). Durch Oxidation der sekundären Alkoholfunktionen in 4-bzw. 8-Stellung geht diese Isomerie verloren. Deshalb existieren von 8-Hydroxy-2,6-dioxabicyclo - [3.3.0]octan-4-on nur noch zwei Isomere, eine 8-endo-und eine 8-exo-Hydroxy-Form. Das zugehörige Diketon, 2,6-Dioxabicyclo[3.3.0]octan-4,8-dion, kommt nur noch in einer Form vor. Entsprechendes gilt für Derivate.

Außer der hier verwendeten Bicyclo-Nomenklatur können die erfindungsgemäßen Verbindungen auch unter der Bezeichnung für anellierte Ringsysteme, als Hexahydro-furo[3.2-b]furane, geführt werden.

Die hier behandelten Verbindungen bestehen deshalb aus den folgenden isomeren Derivaten des 4-Keto-2,6-dioxabicyclo [3.3.0]octan-8-endo-ol-nitrats der allgemeinen Formel I a,

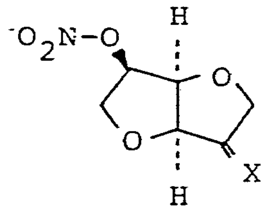

I a

worin X die oben angegebene Bedeutung besitzt, sowie gegebenenfalls deren Salzen und des 4-Keto-2,6-dioxabicyclo[3.3.0]octan-8-exo-ol-nitrats der allgemeinen Formel I b

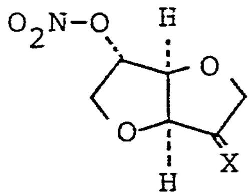

I b

worin X die oben angegebene Bedeutung besitzt, sowie gegebenenfalls deren Salzen.

Ein weiterer Erfindungsgegenstand betrifft Verfahren zur Herstellung von Verbindungen der allgemeinen Formel I. Diese können in an sich bekannter Weise hergestellt werden,

a) dadurch, daß man eines der vorbeschriebenen 4-Keto-2,6-dioxabicyclo[3.3.0]octan-8-ole der Formel II

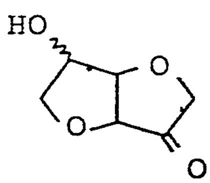

II

worin die OH-Gruppe entweder die endo-oder die exo-Position einnehmen kann, mittels üblicher Methoden in den Nitratester der Formel III überführt,

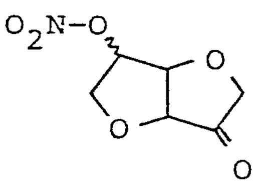

III

worin die $O_2N$-O-Gruppe entweder die endo-oder die exo-Position einnehmen kann, und diesen gegebenenfalls mit Verbindungen $H_2X$ umsetzt, wobei X, mit Ausnahme von O, die oben angegebene Bedeutung besitzt;

b) dadurch, daß man eines der vorbeschriebenen 4-Keto-2,6-dioxabicyclo[3.3.0]octan-8-ole der Formel II, worin die OH-Gruppe entweder die endo-oder die exo-Position einnehmen kann, mittels $H_2X$ in ein Derivat der allgemeinen Formel IV überführt,

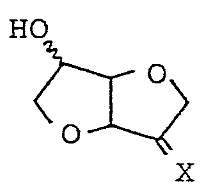

IV

wobei X, mit Ausnahme von O, alle aufgeführten Substituenten darstellt und dieses anschließend auf übliche Weise in den Nitratester umwandelt;

c) dadurch, daß man eines der vorbeschriebenen 2,6-Dioxabicyclo[3.3.0]octan-4,8-diol-mononitrate der Formel V,

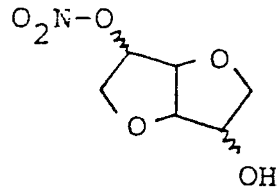

V

worin von der $O_2N$-O-Gruppe und der OH-Gruppe entweder die eine in endo-, die andere in exo-Position stehen oder beide Gruppen entweder gemeinsam die endo-oder die exo-Position einnehmen können, mittels üblicher Methoden oxidiert und das entstandene Keton der Formel III, worin die $O_2N$-O-Gruppe entweder die endo-oder die exo-Position einnehmen kann, gegebenenfalls mit Verbindungen $H_2X$ umsetzt, wobei X, mit Ausnahme von O, die oben angegebene Bedeutung besitzt.

d) Verbindungen der allgemeinen Formel I, in der X entweder einen Rest $(OR^1)_2$ darstellt, worin $R^1$, mit Ausnahme von Methyl und Ethyl, die oben genannte Bedeutung besitzt, oder in der X den Rest

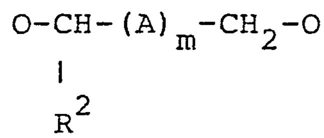

darstellt, worin $R^2$, A und m jeweils die oben genannte Bedeutung besitzen, können auch dadurch hergestellt werden, daß man Verbindungen der allgemeinen Formel I, in der X einen Rest $(OR^1)_2$ darstellt, worin $R^1$ Methyl oder Ethyl bedeutet, mit Alkoholen der allgemeinen Formel VI,

$HOR^1$ VI

worin $R^1$, mit Ausnahme von Methyl und Ethyl, die ange gebene Bedeutung besitzt oder mit Diolen der allgemeinen Formel VII,

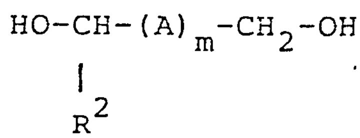

VII

worin $R^2$, A und m jeweils die oben genannte Bedeutung besitzen, einer Umacetalisierung unterwirft.

e) Verbindungen der allgemeinen Formel I, worin X für einen Rest

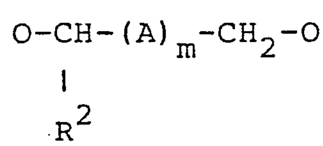

steht, können auch dadurch hergestellt werden, daß man Verbindungen der Formel III mit Dimethylketalen der allgemeinen Formel VIII umsetzt,

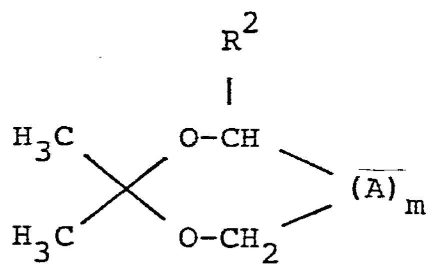

VIII

wobei $R^2$, A und m jeweils die oben angegebene Bedeutung besitzen.

f) Verbindungen der allgemeinen Formel I, worin X für $(OR^1)_2$ steht, können auch dadurch hergestellt werden, daß man Verbindungen der Formel III mit Orthoameisensäure-trialkylestern der allgemeinen Formel IX umsetzt,

$HC(OR^1)_3$ IX

wobei $R^1$ jeweils die oben angegebene Bedeutung besitzt.

Die Herstellung der Nitratester nach den Verfahren a) und b) kann mittels jedem Fachmann geläufigen Methoden erfolgen. Üblicherweise werden hierzu die entsprechenden sekundären Alkohole der Formel II oder IV mittels Nitriersäure (Gemisch aus Salpeter-und Schwefelsäure) oder mittels Salpetersäure, gegebenenfalls in Essigsäure und/oder Essigsäureanhydrid, gelegentlich auch unter Zusatz weiterer Lösungsmittel, umgesetzt und die Nitratester der Formel III oder I auf geeignete Weise isoliert. Die Nitratesterbildung erfolgt dabei im Bereich von -100° C bis + 100° C, bevorzugt zwischen -10° C und + 10° C innerhalb eines Zeitraums von einer Stunde bis zu 24 Stunden.

Selbstverständlich können diese Methoden nur in den Fällen von IV zum Einsatz kommen, in denen X keinen säurelabilen Substituenten darstellt.

Die gegebenenfalls gewünschte Umsetzung von Verbindungen der Formel II oder III mit Verbindungen $H_2X$, worin X, mit Ausnahme von O, die angegebene Bedeutung besitzt, erfolgt, in Abhängigkeit der jeweiligen Bedeutung von X, nach unterschiedlichen, jedoch jedem Fachmann geläufigen Methoden. So werden z.B. die üblichen Verfahren zur Acetalisierung, Oxim-und Hydrazonbildung angewandt, die keiner weiteren Erläuterung bedürfen.

Die Oxidation nach der Verfahrensvariante c) kann sowohl katalytisch als auch durch chemische Oxidationsmittel erfolgen. Bei der katalytischen Dehydrierung gelangt Luft oder Sauerstoff unter Mitwirkung eines geeigneten Katalysators zur Anwendung. Als solche können fungieren: Silber, Platin, Palladium, Kobalt, Nickel, Kupfer, Chrom usw., entweder in reiner Form oder auf einem geeigneten Trägermaterial. Die Reaktion erfolgt gegebenenfalls in einem geeigneten, vom jeweiligen Oxidationsmittel abhängigen Lösungsmittel, wie z.B. Wasser, einem niederen Keton, wie Aceton, Methyl-ethylketon, Diethylketon und dgl., im Bereich zwischen Raumtemperatur und 150° C, vorzugsweise bei der Siedetemperatur des eingesetzten Lösungsmittel und bei Reaktionszeiten zwischen einer und 24 Stunden.

Als chemische Oxidationsmittel können alle diejenigen eingesetzt werden, die in der Lage sind, sekundäre Alkohole in Ketone zu überführen. Dafür kommen insbesondere in Frage: anorganische und organische Chromverbindungen, wie z.B. Alkali-dichromate (Natriumdichromat, Kaliumdichromat), Chromsäure, Pyridinium-dichromat, Pyridiniumchlorochromat, ferner Mangandioxid, Rutheniumtetroxid, Hypohalogenite usw. Dabei werden gewöhnlich, in Abhängigkeit vom eingesetzen Oxidationsmittel, geeignete Lösungsmittel verwendet, wie z.B. Schwefelsäure, Essigsäure, Wasser, Benzol, Toluol, Xylol, Cyclohexan, Dichlormethan, Chloroform, Aceton und dgl. Die Oxidationen können sowohl mit molaren, als auch mit überschüssigen Mengen eines Oxidationsmittels erfolgen und werden üblicherweise in einem Temperaturbereich zwischen 0° C und dem Siedepunkt des eingesetzten Lösungsmittels und bei Reaktionszeiten zwischen einer und 24 Stunden durchgeführt.

Eine ausführliche Darstellung geeigneter Oxidationsmethoden liegt vor in: Houben-Weyl, Methoden der Organischen Chemie, Bd. VII/2 a, S. 699 ff, Thieme Verlag, 1973.

Die Umacetalisierung nach Methode d) erfolgt in einer jedem Fachmann geläufigen Weise, durch Behandeln des Dimethyl-oder Diethylacetals der allgemeinen Formel I, worin X einen Bis-alkoxy-Rest $(OR^1)_2$ darstellt, in dem $R^1$ Methyl oder Ethyl bedeutet, mit Alkoholen der allgemeinen Formel VI bzw. mit Diolen der allgemeinen Formel VII. Dabei kann der Alkohol VI und das Diol VII entweder nur als Reaktionspartner oder als solcher und gleichzeitig als Lösungsmittel zum Einsatz kommen. Im ersten Fall wird man mit ungefähr molaren Mengen auskommen und gegebenenfalls ein geeignetes, unter den Reaktionsbedingungen inertes Lösungsmittel zusetzen. Als solche können verwendet werden: Benzol, Toluol, Xylol, Diethylether, Diisopropylether, Tetrahydrofuran, Dioxan, Dichlormethan, Dichlorethan, Chloroform und dgl.. Im zweiten Fall arbeitet man mit einem Überschuß an Alkohol VI bzw. Diol VII. Die Reaktion erfolgt in beiden Fällen im allgemeinen zwischen Raumtemperatur und 150° C, im besonderen zwischen 50° C und der

Siedtemperatur des eingesetzten Alkohols, Diols oder Lösungsmittels. Durch Zusatz eines geeigneten Umacetalisierungskatalysators kann die Reaktion beschleunigt werden. Als solche kommen im Frage: anorganische Mineralsäuren wie z.B. Schwefelsäure, Chlorwasserstoffsäure, Bromwasserstoffsäure und Phosphorsäure, organische Carbon-und Sulfonsäuren wie z.b. Benzoesäure, Benzolsulfonsäure, Toluolsulfonsäure und Camphersulfonsäure, sowie andere saure Katalysatoren wie z.B. Kaliumhydrogensulfat, Eisen-(III) chlorid, Bortrifluorid usw. Sie ist im allgemeinen zwischen einer und 50 Stunden beendet.

Eine zusammenfassende Darstellung der Herstellung von Acetalen, einschließlich der Umacetalisierung findet sich in Houben-Weyl, Methoden der Organischen Chemie, Bd. VI/3, S. 199 ff., Thieme Verlag, 1965.

Die Umacetalisierung nach der Methode e) erfolgt in an sich bekannter Weise durch Umsetzung von Ketonen der Formel III mit Dimethylketalen der allgemeinen Formel VIII. Hierbei entsprechen die Reaktionsbedingungen den unter d) angegebenen.

Für die Reaktion gemäß Variante f) werden Verbindungen der Formel III in an sich bekannter Weise mit Orthoameisensäure-trialkylestern der allgemeinen Formel IX umgesetzt. Auch in diesem Fall kamm der Orthoameisensäure-trialkylester entweder nur als Reaktionspartner oder als solcher und gleichzeitig als Lösungsmittel zum Einsatz kommen. Im ersteren Fall wird man mit ungefähr molaren Mengen auskommen und gegebenenfalls ein geeignetes Lösungsmittel zusetzen. Im zweite Fall arbeitet man mit einem Überschuß. Als zweckmäßig hat sich in der Praxis auch der Zusatz eines den Alkylgruppen des Orthoameisensäure-trialkylesters entsprechenden Alkohols erwiesen. Die Reaktion erfolgt im allgemeinen zwischen 0° C und dem Siedepunkt des eingesetzten Alkohols, Orthoameisensäuretrialkylesters oder Lösungsmittels, bevorzugt jedoch bei Raumtemperatur und erfordert Reaktionszeiten zwischen einer und 50 Stunden. Durch Zusatz eines geeigneten Katalysators kann die Reaktion beschleunigt werden. Als solche kommen in Frage: anorganische Mineralsäuren, wie z.B. Schwefelsäure, Chlorwasserstoffsäure, Bromwasserstoffsäure und Phosphorsäure, organische Carbon-und Sulfonsäuren, wie z.B. Benzoesäure, Oxalsäure, Essigsäure, Benzolsulfonsäure, Toluolsulfonsäure und Camphersulfonsäure, sowie andere saure Katalysatoren wie z.B. Kaliumhydrogensulfat, Eisen(III)chlorid, Ammoniumchlorid, Ammoniumnitrat und die Hydrochloride von Mono-, Di-und Triethylamin und des Pyridins.

Eine Zusammenfassung dieser Methoden findet sich ebenfalls in der bereits zitierten Literatur in Houben-Weyl, Bd.VI/3.

Die erfindungsgemäßen Verbindungen zeichnen sich überraschenderweise durch bemerkenswerte pharmakologische Eigenschaften und gute Verträglichkeit aus und stellen deshalb wertvolle Arzneistoffe dar. Insbesondere können sie zur Prophylaxe und Therapie von Herz-und Kreislauferkrankungen eingesetzt werden, wie z.B. Angina Pectoris, Herzinfarkt, Bluthochdruck und dgl. Durch ihr neuartiges Wirkungsprofil unterscheiden sie sich von anderen bekannten Arzneimitteln dieses Indikationsgebietes und stellen somit eine Bereicherung der Pharmazie dar.

Gegenstand der Erfindung ist deshalb auch die Verwendung von Verbindungen der allgemeinen Formel I als Arzneimittel. Derartige Arzneimittel können die erfindungsgemäßen Verbindungen in einem Gehalt von 0,1 bis 99,9 % enthalten. Die Dosierung richtet sich nach dem vorgesehenen Einsatzzweck und der Schwere der Erkrankung und kann wie gewünscht gewählt werden. Üblicherweise liegt sie im Bereich von 1 mg bis 500 mg, wobei die Verabreichung einmal oder mehrmals täglich erfolgen kann. Als pharmazeutische Darreichungsformen kommen alle, dem Fachmann geläufigen Formulierungen in Frage, wie z.B. Pulver, Granulate, Tabletten, Kapseln, Suppositorien, Suspensionen, Liquida, Injectabilia und transdermale Systeme. Zur Herstellung pharmazeutischer Darreichungsformen können feste, halbfeste oder flüssige Trägermaterialien oder Verdünnungsmittel eingesetzt werden. Darin sind eingeschlossen Corrigentien, Bindemittel, Gleitmittel, Emulgatoren usw. Beispiele für derartige Mittel sind Stärke, wie Kartoffel-und Getreidestärke, Zucker, wie Lactose, Sucrose, Glucose, Mannitol, Sorbitol, Cellulose, wie kristalline Cellulose, Methyl-cellulose, Calcium-carboxymethyl-cellulose, Natrium-carboxymethyl-cellulose und Hydroxypropyl-cellulose, anorganische Materialien wie Kaliumphosphat, Calciumsulfat, Calcium carbonat und Talkum, Gelatine, Gummiarabicum, Polyvinylpyrrolidon, oberflächenaktive Substanzen wie Fettsäureglyceride, Fettsäure-sorbitanester, Fettsäureester von Sucrose, Polyglycerol und andere.

Einige Beispiele für Arzneimittelformulierungen, unter Benutzung der erfindungsgemäßen Verbindungen, sind nachfolgend aufgeführt:

Tabletten:

| Zusammensetzung | mg/Tablette |
|---|---|
| erfindungsgemäße Verbindung | 3 |
| mikrokristalline Cellulose | 25 |
| Lactose | 17 |
| Carboxymethylcellulose-Calcium | 4,5 |
| Magnesiumstearat | 0,5 |

Obige Ingredienzien werden gesiebt, sorgfältig und ausreichend gemischt und auf einer geeigneten Tablettenpresse gepreßt.

Kapseln:

| Zusammensetzung | mg/Kapsel |
|---|---|
| erfindungsgemäße Verbindung | 10 |
| Lactose | 40 |
| mikrokristalline Cellulose | 30 |
| Talkum | 10 |

Obige Ingredienzien werden gesiebt, sorgfältig und ausreichend gemischt und auf einer geeigneten Kapselfüllmaschine in Hartgelatinekapseln gefüllt.

Gegenstand der Erfindung ist auch die Verwendung der erfindungsgemäßen Verbindungen der allgemeinen Formel I bei der Vorbeugung und Bekämpfung von Krankheiten, insbesondere von Herz-und Kreislauferkrankungen.

Die nachfolgenden Beispiele dienen der Erläuterung der Erfindung.

Beispiel 1

4-Oxo-2,6-dioxabicyclo[3.3.0]octan-8-endo-ol-nitrat

Zu einer aus 13,0 g 65-proz. Salpetersäure und 40 ml Acetanhydrid unter Kühlen bereiteten Nitriermischung gibt man portionsweise, unter Rühren, bei 5-10° C, 14,4 g 4-Oxo-2,6-dioxabicyclo[3.3.0]octan-8-endo-ol [Chem. Ber. 96, 3195 (1963)]. Man rührt noch 1 Stunde im Eisbad und danach 10 Stunden bei Raumtemperatur. Anschließend gießt man in ca. 200 ml Eiswasser und extrahiert mehrmals mit Dichlormethan. Die vereinigten organischen Phasen werden vorsichtig mit verdünnter wäßriger Natriumcarbonatlösung, danach mit gesättigter Natriumchloridlösung neutral gewaschen, getrocknet und am Rotavapor eingeengt. Der Rückstand wird aus Diisopropylether umkristallisiert.

Schmp. 59-62° C, $[\alpha]_D^{20}$ + 212,6° (c = 0,896, Aceton).

$C_6H_7NO_6$ (189,13).

Beispiel 2

4-Oxo-2,6-dioxabicyclo[3.3.0]octan-8-exo-ol-nitrat

64,7 g Pyridiniumchlorochromat werden in 500 ml Benzol suspendiert und unter kräftigem Rühren zum Sieden erhitzt. Dazu gibt man eine kochende Lösung von 19,1 g 2,6-Dioxabicyclo[3.3.0]octan-4-(exo), 8-(endo)-diol-4-nitrat (Isosorbid-2-nitrat) in 330 ml Benzol. Es erfolgt eine heftige exotherme Reaktion, und am eingebauten Wasserabscheider beginnt sich das gebildete Reaktionswasser zu sammeln. Nachdem man

noch 1,5 Stunden unter Rückfluß erhitzt hat, wird heiß über Celite abgesaugt und der schwarze Rückstand noch 3 x mit je 100 ml Benzol ausgekocht. Die vereinigten Benzolphasen filtiert man über eine kurze, mit Kieselgel gefüllte Säule, wobei gefärbte Produkte vollständig zurückgehalten werden. Danach engt man auf ein Volumen von ca. 15 ml ein und läßt das Produkt unter Kühlung auskristallisieren.

Schmp. 69-72° C, $[\alpha]_D^{20}$ + 80,3° (c = 0,691, Aceton).

$C_6H_7NO_6$ (189,13)

Beispiel 3

4-Hydroximino-2,6-dioxabicyclo[3.3.0]octan-8-endo-ol-nitrat

In eine Lösung von 6,95 g Hydroxylammoniumchlorid in 120 ml Wasser trägt man zur Freisetzung der Base 6,9 g Kaliumcarbonat ein. Dazu tropft man unter Rühren eine Lösung von 18,9 g 4-Oxo-2,6-dioxabicyclo[3.3.0]octan-8-endo-ol-nitrat in 200 ml Methanol. Man läßt 3 Stunden bei Raumtemperatur reagieren und destilliert anschließend das Methanol i.Vak. ab. Die zurückbleibende Wasserphase wird mit Natriumchlorid gesättigt und 3 x mit Chloroform extrahiert. Die vereinigten organischen Phasen werden getrocknet und eingeengt, der Rückstand aus Diisobutylether umkristallisiert.

Schmp. 111-115° C, $[\alpha]_D^{20}$ + 280,4° (c = 0,929, Methanol).

$C_6H_8N_2O_6$ (204,15)

Beispiel 4

4-Hydroximino-2,6-dioxabicyclo[3.3.0]octan-8-exo-ol-nitrat

18,9 g 4-Oxo-2,6-dioxabicyclo[3.3.0]octan-8-exo-ol-nitrat werden mit 6,95 g Hydroxylammoniumchlorid analog Beispiel 3 umgesetzt und das Reaktionsprodukt aus Toluol umkristallisiert.

Schmp. 113-115° C, $[\alpha]_D^{20}$ + 114,6° (c = 0,825, Methanol).

$C_6H_8N_2O_6$ (204,15)

Beispiel 5

4-Semicarbazono-2,6-dioxabicyclo[3.3.0]octan-8-endo-ol-nitrat

9,5 g 4-Oxo-2,6-dioxabicyclo[3.3.0]octan-8-endo-ol-nitrat, 5,6 g Semicarbazid-hydrochlorid und 8,2 g Natriumacetat werden in 80 ml Wasser gelöst. Man erwärmt kurz auf 50-60° C, wobei ein farbloser Niederschlag ausfällt. Dieser wird nach dem Abkühlen abfiltriert und aus Methanol umkristallisiert.

Schmp. 190° C.

$C_7H_{10}N_4O_6$ (246,19)

Beispiel 6

4-Semicarbazono-2,6-dioxabicyclo[3.3.0]octan-8-exo-ol-nitrat

9,5 g 4-Oxo-2,6-dioxabicyclo[3.3.0]octan-8-exo-ol-nitrat werden mit 5,6 g Semicarbazid-hydrochlorid und 8,2 g Natriumacetat analog Beispiel 5 umgesetzt und das Reaktionsprodukt aus Methanol umkristallisiert.

Schmp. 196-197° C (Zers.), $[\alpha]_D^{20}$ + 87,8° (c = 0,564, DMF).

$C_7H_{10}N_4O_6$ (246,19)

Beispiel 7

4,4-Dimethoxy-2,6-dioxabicyclo[3.3.0]octan-8-endo-ol-nitrat

18,9 g 4-Oxo-2,6-dioxabicyclo[3.3.0]octan-8-endo-ol-nitrat werden mit 21,2 g Orthoameisensäure-trimethylester und einer katalytischen Menge p-Toluolsulfonsäure in 200 ml wasserfreiem Methanol 20 Stunden bei Raumtemperatur gerührt. Danach engt man i.Vak. ein, nimmt den Rückstand in Dichlormethan auf und schüttelt 2 x mit Wasser aus. Der nach dem Einengen der organischen Phase verbleibende Rückstand wird aus Methanol umkristallisiert.

Schmp. 103-104° C, $[\alpha]_D^{20}$ + 214,7° (c = 0,927, Aceton).
$C_8H_{13}NO_7$ (235,19)

Beispiel 8

4,4-Dimethoxy-2,6-dioxabicyclo[3.3.0]octan-8-exo-ol-nitrat

Die Herstellung erfolgt analog Beispiel 7, aus 18,9 g 4-Oxo-2,6-dioxabicyclo[3.3.0]octan-8-exo-ol-nitrat und 21,2 g Orthoameisensäure-trimethylester in 200 ml wasserfreiem Methanol. Der ölige Rückstand wird i.Vak. destilliert.

Sdp. 84-85° C / 0,2 Torr, $[\alpha]_D^{20}$ + 8,10° (c = 0,73, Aceton).
$C_8H_{13}NO_7$ (235,19)

Beispiel 9

4,4-Diethoxy-2,6-dioxabicyclo[3.3.0]octan-8-endo-ol-nitrat

Die Herstellung erfolgt analog. Beispiel 7, aus 18,9 g 4-Oxo-2,6-dioxabicyclo[3.3.0]octan-8-endo-ol-nitrat und 16,3 g Orthoameisensäure-triethylester in 200 ml wasserfreiem Ethanol.

Schmp. 44-45° C (aus n-Heptan), $[\alpha]_D^{20}$ + 185,3° (c = 0,619, Aceton).
$C_{10}H_{17}NO_7$ (263,25)

Beispiel 10

4,4-Diethoxy-2,6-dioxabicyclo[3.3.0]octan-8-exo-ol-nitrat

Die Herstellung erfolgt analog Beispiel 7, aus 18,9 g 4-Oxo-2,6-dioxabicyclo[3.3.0]octan-8-exo-ol-nitrat und 16,3 g Orthoameisensäure-triethylester in 200 ml wasserfreiem Ethanol. Der ölige Rückstand wird i.Vak. destilliert.

Sdp. 85-87° C / 0,2 Torr, $[\alpha]_D^{20}$ + 6,10° (c = 0,978, Aceton).
$C_{10}H_{17}NO_7$ (263,25)

Beispiel 11

4,4-Ethylendioxy-2,6-dioxabicyclo-[3.3.0]octan-8-endo-ol-nitrat

9,45 g 4-Oxo-2,6-dioxabicyclo[3.3.0]octan-8-endo-ol-nitrat werden mit 80 ml Ethylenglykol und 3 ml konz. Salzsäure 2 Tage bei 70° C gerührt. Danach wird der Ansatz mit ca. 100 ml Wasser verdünnt und 3 x mit Dichlormethan extrahiert. Die vereinigten organischen Phasen werden mit gesättigter wäßriger Natriumchloridlösung gewaschen, getrocknet und eingeengt, der Rückstand aus Diisopropylether umkristallisiert.

Schmp. 100-101° C, $[\alpha]_D^{20}$ + 206,7° (c = 0,716, Aceton).
$C_8H_{11}NO_7$ (233,18)

Beispiel 12

4,4-Ethylendioxy-2,6-dioxabicyclo[3.3.0]octan-8-exo-ol-nitrat

13,16 g 4,4-Diethoxy-2,6-dioxabicyclo[3.3.0]octan-8-exo-ol-nitrat (Beispiel 10) werden mit 50 ml Ethylenglykol und einer katalytischen Menge p-Toluolsulfonsäure 5 Stunden bei 100° im Wasserstrahlvakuum gerührt. Danach wird der Ansatz mit 100 ml Wasser verdünnt und 2 x mit Dichlormethan extrahiert. Die organische Phase wird getrocknet und i. Vak. eingeengt, der ölige Rückstand i.Vak. destilliert.

Sdp. 101-102° C / 0,15 Torr, $[\alpha]_D^{20}$ + 67,20° (c = 0,96, Aceton).

$C_8H_{11}NO_7$ (233,18)

Beispiel 13

4,4-(1,3-Propylendioxy)-2,6-dioxabicyclo[3.3.0]octan-8-endo-ol-nitrat

Die Herstellung erfolgt analog Beispiel 12, aus 13,16 g 4,4-Diethoxy-2,6-dioxabicyclo[3.3.0]octan-8-endo-ol-nitrat (Beispiel 9) und 50 ml 1,3-Propandiol, unter Zusatz einer katalytischen Menge p-Toluolsulfonsäure.

Schmp. 85° C (aus Diisopropylether), $[\alpha]_D^{20}$ + 216,3° (c = 0,735, Aceton).

$C_9H_{13}NO_7$ (247,21)

Beispiel 14

4,4-(1,3-Propylendioxy)-2,6-dioxabicyclo[3.3.0]octan-8-exo-ol-nitrat

Die Herstellung erfolgt analog Beispiel 12, aus 13,16 g 4,4-Diethoxy-2,6-dioxabicyclo[3.3.0]octan-8-exo-ol-nitrat (Beispiel 10) und 50 ml 1,3-Propandiol, unter Zusatz einer katalytischen Menge p-Toluolsulfonsäure. Der Rückstand wird zunächst i.Vak. destilliert und das erstarrte Destillat anschließend aus Diisopropylether umkristallisiert.

Sdp. 112-115° C / 0,2 Torr, Schmp. 70-71° C, $[\alpha]_D^{20}$ + 78,20° (c = 0,844, Aceton).

$C_9H_{13}NO_7$ (247,21)

Beispiel 15

4,4-Bis-(octadecanyloxy)-2,6-dioxabicyclo[3.3.0]octan-8-exo-ol-nitrat

5,3 g 4,4-Diethoxy-2,6-dioxabicyclo[3.3.0]octan-8-exo-ol-nitrat (Beispiel 10) werden mit 27,0 g 1-Octadecanol und einer katalytischen Menge p-Toluolsulfonsäure 5 Stunden bei 80° C im Wasserstrahlvakuum gerührt. Überschüssiger Stearylalkohol wird mehrmals mit heißem Methanol von einem darin unlöslichen, öligen Rückstand extrahiert. Dieser Rückstand erstarrt beim Abkühlen und wird aus Isopropanol umkristallisiert.

Schmp. 47° C, $[\alpha]_D^{20}$ + 29,6° (c = 0,675, Chloroform).

$C_{42}H_{81}NO_7$ (712,01)

Beispiel 16

4,4-Bis-(2,2-dimethyl-1-propoxy)-2,6-dioxabicyclo[3.3.0] octan-8-exo-ol-nitrat

13,2 g 4,4-Diethoxy-2,6-dioxabicyclo[3.3.0]octan-8-exo-ol-nitrat (Beispiel 10) werden mit 44,0 g 2,2-Dimethyl-1-propanol und einer katalytischen Menge p-Toluolsulfonsäure 5 Stunden bei 100° C und einem Vakuum von ca. 500 Torr gerührt. Nach dem Abkühlen verdünnt man mit 200 ml Dichlormethan, wäscht 1 x mit Wasser, 2 x mit verdünnter wäßriger Natriumcarbonatlösung, danach wieder 2 x mit Wasser und entfernt das Lösungsmittel sowie überschüssiges 2,2-Dimethyl-1-propanol am Rotavapor i.Vak.. Der Rückstand wird aus Methanol umkristallisiert.

Schmp. 104-105° C, $[\alpha]_D^{20}$ + 37,4° (c = 0,816, Aceton).
$C_{16}H_{29}NO_7$ (347,41)

Beispiel 17

4-Thiosemicarbazono-2,6-dioxabicyclo[3.3.0]octan-8-exo-ol-nitrat

Zwei Lösungen aus 18,9 g 4-Oxo-2,6-dioxabicyclo[3.3.0] octan-8-exo-ol-nitrat in 100 ml Ethanol und 9,1 g Thiosemicarbazid in 500 ml Ethanol werden vereinigt und 1 Stunde bei Raumtemperatur gerührt, wobei ein Niederschlag ausfällt. Dieser wird abgesaugt und aus Methanol umkristallisiert.
Schmp. 189-190° C, $[\alpha]_D^{20}$ + 100,7° (c = 0,68, Aceton).
$C_7H_{10}N_4O_5S$ (262,24)

Beispiel 18

4-Amidinohydrazono-2,6-dioxabicyclo[3.3.0]octan-8-exo-ol-nitrat

Eine Lösung von 3,8 g 4-Oxo-2,6-dioxabicyclo[3.3.0]octan-8-exo-ol-nitrat in 100 ml Ethanol wird mit 2,7 g Aminoguanidin-hydrogencarbonat versetzt. Zu dieser Suspension tropft man unter Rühren bei Raumtemperatur langsam 20 ml konz. Salzsäure. Zunächst entsteht eine klare Lösung, aus der nachfolgend ein farbloser Niederschlag ausfällt. Dieser wird nach 1 Stunde abgesaugt, mit Eiswasser gewaschen und aus Ethanol umkristallisiert.
Hydrochlorid: Schmp. 196° C (Zers.), $[\alpha]_D^{20}$ + 122,8° (c = 0,338, Wasser).
$C_7H_{11}N_5O_5$ . HCl (281,66)

Beispiel 19

4-($\Delta^2$-Imidazolin-2-yl-hydrazono)-2,6-dioxabicyclo[3.3.0] octan-8-exo-ol-nitrat

3,8 g 4-Oxo-2,6-dioxabicyclo[3.3.0]octan-8-exo-ol-nitrat und 3,6 g 2-Hydrazino-2-imidazolin-hydrobromid werden in 200 ml Ethanol gelöst und 15 Stunden bei Raumtemperatur gerührt. Danach wird das Lösungsmittel i.Vak. abdestilliert, der Rückstand in Wasser aufgenommen und alkalisiert. Dabei fällt die Base aus, die man absaugt und zunächst aus Methanol, danach aus Wasser umkristallisiert.
Schmp. 189-190° C (Zers.), $[\alpha]_D^{20}$ + 170,5° (c = 0,789, Aceton).
$C_9H_{13}N_5O_5$ (271,23)

Beispiel 20

4,4-(4-Nitrooxymethyl-ethylendioxy)-2,6-dioxabicyclo[3.3.0] octan-8-exo-ol-nitrat

3,8 g 4-Oxo-2,6-dioxabicyclo[3.3.0]octan-8-exo-ol-nitrat werden mit 2,7 g Glycerin-1-nitrat in 200 ml Benzol unter Zusatz einer katalytischen Menge p-Toluolsulfonsäure 8 Stunden am Wasserabscheider unter Rückfluß erhitzt. Nach dem Abkühlen schüttelt man 3 x mit je 50 ml Wasser aus, trocknet die Benzolphase und engt i.Vak. ein. Der Rückstand wird über eine Kieselgelsäule mit Chloroform/Methanol 98:2 chromatografiert.
Farbloses Öl, $[\alpha]_D^{20}$ + 68,7° (c = 1,186, Methanol), Rf = 0,56
(DC-Fertigplatte, Kieselgel 60, Fluka, Laufmittel Chloroform/Methanol 99/1).
$C_9H_{12}N_2O_{10}$ (308,21)

Beispiel 21

4,4-(4-Chlormethyl-ethylendioxy)-2,6-dioxabicyclo[3.3.0] octan-8-exo-ol-nitrat

3,8 g 4-Oxo-2,6-dioxabicyclo[3.3.0]octan-8-exo-ol-nitrat werden mit 2,2 g 3-Chlor-1,2-propandiol in 150 ml Benzol, unter Zusatz einer katalytischen Menge p-Toluolsulfonsäure, 12 Stunden am Wasserabscheider unter Rückfluß erhitzt. Nach dem Abkühlen schüttelt man 3 x mit je 50 ml Wasser aus, trocknet die Benzolphase und engt i.Vak. ein. Der Rückstand wird über eine Kieselgelsäule mit Chloroform/Methanol 99:1 chromatografiert.

Farbloses Öl, $[\alpha]_D^{20}$ + 65,9° (c = 0,888, Methanol), Rf = 0,53 (DC-Fertigplatte Kieselgel 60, Fluka, Laufmittel Chloroform/Methanol 99/1).

$C_9H_{12}ClNO_7$ (281,65)

Beispiel 22

4-Ethylcarbazono -2,6-dioxabicyclo[3.3.0]octan-8-exo-ol-nitrat

Eine Lösung von 9,5 g 4-Oxo-2,6-dioxabicyclo[3.3.0]octan-8-exo-ol-nitrat in 150 ml Dichlormethan und eine Lösung von 5,2 g Hydrazincarbonsäureethylester in 150 ml Wasser werden vereinigt und bei Raumtemperatur 45 Min. kräftig gerührt. Danach trennt man die wäßrige Phase ab und schüttelt die organische Phase zunächst mit 100 ml 0,01 n Salzsäure, anschließend 3 x mit je 100 ml 0,01 n Natriumhydroxidlösung aus. Der nach dem Trocknen und Einengen verbleibende Rückstand stellt das Gemisch der syn- und anti-Form der Titelverbindung dar.

Farbloses Öl, $[\alpha]_D^{20}$ + 80,8° (c = 1,114, Methanol), Rf = 0,53/0,80 (DC-Fertigplatte Kieselgel 60, Fluka, Laufmittel Chloroform/Methanol 95/5).

$C_9H_{13}N_3O_7$ (275,22)

Beispiel 23

4,4-(4-Hydroxymethyl-ethylendioxy)-2,6-dioxabicyclo[3.3.0] octan-8-exo-ol-nitrat

9,5 g 4-Oxo-2,6-dioxabicyclo[3.3.0]octan-8-exo-ol-nitrat werden mit 66,0 g 1,2-Isopropyliden-glycerol und einer katalytischen Menge p-Toluolsulfonsäure 8 Stdn. bei 130° im Ölbad gerührt. Nach dem Abkühlen verdünnt man mit 500 ml Wasser und extrahiert mit Methylenchlorid. Der Extrakt wird getrocknet, am Rotationsverdampfer i.Vak. eingeengt, und der Rückstand über eine Kieselgelsäule mit Methylenchlorid/Ether 1:1 chromatografisch gereinigt. Man erhält auf diese Weise ein Isomerengemisch als schwach gelbliches Öl, aus dem sich nach einiger Zeit Kristalle abscheiden. Diese werden durch Umkristallisieren aus Diisopropylether gereinigt.

Schmp. 129-130° C (Isomerengemisch), $[\alpha]_D^{20}$ + 82,9° (c = 0,802, Methanol).

$C_9H_{13}NO_8$ (263,21)

## Ansprüche

1. 2,6-Dioxabicyclo[3.3.0]octan-Derivate der allgemeinen Formel I,

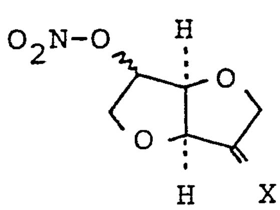

I

worin die Bindung zwischen Ringsystem und $O_2N$-O-Gruppe sowohl endo-als auch exo-cyclisch angeordnet

sein kann und worin X für einen der nachfolgenden Substituenten steht:

$$O$$

$$(OR^1)_2$$

wobei $R^1$ einen geradkettigen oder verzweigten Alkylrest mit 1 bis 20 Kohlenstoffatomen darstellt,

$$\begin{matrix} O-CH-(A)_m-CH_2-O \\ | \\ R^2 \end{matrix}$$

wobei $R^2$ Wasserstoff oder einen $-CH_2$-Hal, $-CH_2-OH$ oder $-CH_2-ONO_2$ Rest, A einen $=CH_2$, $=CH$-Hal, $=CH-OH$ oder $=CH-ONO_2$ Rest und m die Zahlen 0 oder 1 bedeuten, wobei Hal für einen Halogenrest, insbesondere für einen Chlor-, Brom-oder Jodrest steht,

$$N-OH$$

$$N-NH-R^3$$

wobei $R^3$ Wasserstoff oder einen der Reste

$$\begin{matrix} C=Y, \\ | \\ NH_2 \end{matrix}$$

wobei Y für O, S oder NH steht, oder
$COOR^4$, wobei $R^4$ für eine geradkettige oder verzweigte Alkylgruppe mit 1 bis 5 Kohlenstoffatomen steht oder

$$\begin{matrix} C & = & N, \\ | & & | \\ NH- & (CH_2) & _n \end{matrix}$$

wobei n die Zahlen 1 bis 4 bedeuten,
sowie gegebenenfalls deren Salze mit anorganischen oder organischen Säuren.

2. 2,6-Dioxabicyclo[3.3.0]octan-Derivate gemäß Anspruch 1, dadurch gekennzeichnet, daß sie Derivate des 2,6-Dioxabicyclo[3.3.0]octan-8-endo-ol-nitrats der allgemeinen Formel I a sind,

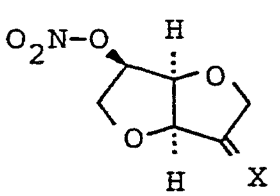

I a

worin X die oben angegebene Bedeutung besitzt.

3. 2,6-Dioxabicyclo[3.3.0]octan-Derivate gemäß Anspruch 1, dadurch gekennzeichnet, daß sie Derivate des 2,6-Dioxabicyclo[3.3.0]octan-8-exo-ol-nitrats der allgemeinen Formel I b sind

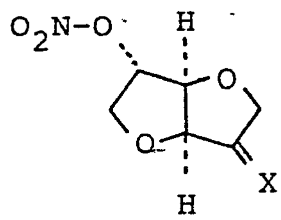

I b

worin X die oben angegebene Bedeutung besitzt.

4. Verfahren zur Herstellung von 2,6-Dioxabicyclo[3.3.0] octan-Derivaten gemäß Anspruch 1, dadurch gekennzeich net, daß man in an sich bekannter Weise:

a) 4-Keto-2,6-dioxabicyclo[3.3.0]octan-8-ole der Formel II

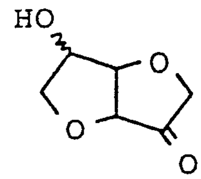

II

worin die OH-Gruppe entweder die endo-oder die exo-Position einnehmen kann, mittels üblicher Methoden in die Nitratester der Formel III überführt,

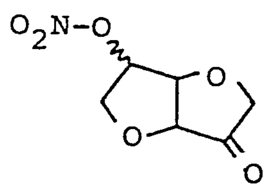

III

worin die $O_2N$-O-Gruppe entweder die endo-oder die exo-Position einnehmen kann, und diese gegebenenfalls mit Verbindungen $H_2X$ umsetzt, wobei X, mit Ausnahme von O, die oben angegebene Bedeutung besitzt;

b) 4-Keto-2,6-dioxabicyclo[3.3.0]octan-8-ole der Formel II, worin die OH-Gruppe entweder die endo-oder die exo-Position einnehmen kann, mittels $H_2X$ in ein Derivat der allgemeinen Formel IV überführt,

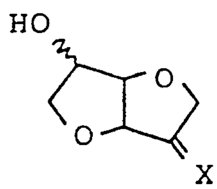

IV

worin X, mit Ausnahme von O, alle aufgeführten Substituenten darstellt und dieses anschließend auf übliche Weise in den Nitratester umwandelt;

c) 2,6-Dioxabicyclo[3.3.0]octan-4,8-diolmononitrate der Formel V

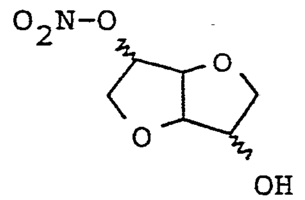

V

worin von der $O_2N$-O-Gruppe und der OH-Gruppe entweder die eine in endo-, die andere in exo-Position stehen oder beide Gruppen entweder gemeinsam die endo-oder die exo-Position einnehmen können, mittels üblicher Methoden oxidiert und das entstandene Keton der Formel III, worin die $O_2N$-O-Gruppe entweder die endo-oder die exo-Position einnehmen kann, gegebenenfalls mit Verbindungen $H_2X$ umsetzt, wobei X, mit Ausnahme von O, die oben angegebene Bedeutung besitzt;

d) zur Herstellung von Verbindungen der allgemeinen Formel I, in der X entweder einen Fest $(OR^1)_2$ darstellt, worin $R^1$, mit Ausnahme von Methyl und Ethyl, die oben genannte Bedeutung besitzt, oder in der X den Rest

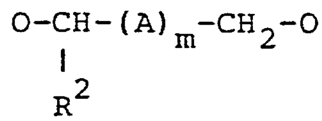

darstellt, in dem $R^2$, A und m die oben genannte Bedeutung besitzen, Verbindungen der allgemeinen Formel I, worin X einen Rest $(OR^1)_2$ darstellt, in dem $R^1$ Methyl und Ethyl bedeutet, mit Alkoholen der allgemeinen Formel VI,

$HOR^1$ VI

worin $R^1$, mit Ausnahme von Methyl und Ethyl, die angegebene Bedeutung besitzt oder mit Diolen der allgemeinen Formel VII

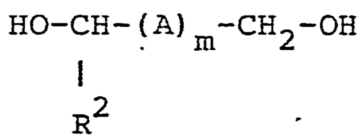

VII

worin $R^2$, A und m die oben genannte Bedeutung besitzt, einer Umacetalisierung unterwirft;

e) zur Herstellung von Verbindungen der allgemeinen Formel I, worin X für den Rest

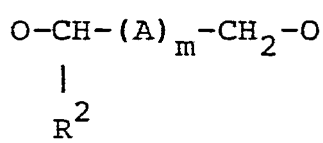

steht, Verbindungen der Formel III mit Dimethylketalen der allgemeinen Formel VIII umsetzt,

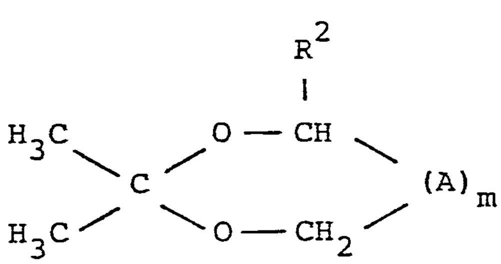

VIII

wobei $R^2$, A und m jeweils die oben angegebene Bedeutung besitzen;

f) zur Herstellung von Verbindungen der allgemeinen Formel I, worin X für $(OR^1)_2$ steht, Verbindungen der Formel III mit Ortho-ameisensäure-trialkylestern der allgemeinen Formel IX umsetzt,
$HC(OR^1)_3$ IX
wobei $R^1$ jeweils die oben angegebene Bedeutung besitzt.

5. 2,6-Dioxabicyclo[3.3.0]octan-Derivate gemäß Anspruch 1 zur Verwendung als Arzneimittel.

6. Verwendung von 2,6-Dioxabicyclo[3.3.0]octan-Derivaten gemäß Anspruch 1 bei der Bekämpfung und Prophylaxe von Krankheiten, insbesondere von Herz-und Kreislauferkrankungen.

7. Arzneimittel enthaltend mindestens eine Verbindung der allgemeinen Formel I gemäß Anspruch 1 und gegebenenfalls übliche Hilfs-und Trägerstoffe.